# EUROPÄISCHE PATENTSCHRIFT

(11) **EP 0 787 002 B1**
(45) Veröffentlichungstag und Bekanntmachung des Hinweises auf die Patenterteilung: **13.12.2006**
(21) Anmeldenummer: 95937845.6
(22) Anmeldetag: 24.10.1995
(51) Int. Cl.: A61K 31/56, A61K 31/565, A61K 31/575, A61P 15/18

(54) **KOMPETITIVE PROGESTERONANTAGONISTEN ZUR BEDARFSORIENTIERTEN WEIBLICHEN FERTILITÄTSKONTROLLE**
COMPETITIVE PROGESTERONE ANTAGONISTS FOR REGULATING FEMALE FERTILITY AS REQUIRED
ANTAGONISTES COMPETITIFS DE LA PROGESTERONE PERMETTANT DE REGULER A LA DEMANDE LA FECONDITE FEMININE

(30) Priorität: 24.10.1994 DE 4438820
(43) Veröffentlichungstag der Anmeldung: 06.08.1997
(73) Patentinhaber: Schering Aktiengesellschaft, 13342 Berlin (DE)
(72) Erfinder: CHWALISZ, Kristof, D-13503 Berlin (DE); STÖCKEMANN, Klaus, D-12161 Berlin (DE); SCHMIDT-GOLLWITZER, Karin, D-14129 Berlin (DE); KLEMANN, Walter, 1428 Buenos Aires (AR)
(86) Internationale Anmeldenummer: PCT/EP1995/004191
(87) Internationale Veröffentlichungsnummer: WO 1996/012494

(56) Entgegenhaltungen:
- WO-A-93/23020
- EXPERIMENTAL AND CLINICAL ENDOCRINOLOGY, Bd. 81, Nr. 2, 1983 Seiten 122-136, STRECKE, J. ET AL 'Interceptive activities of STS 557 in rabbits, mice and rats'
- JOURNAL OF STEROID BIOCHEMISTRY, Bd. 11, Nr. 1C, 1979 Seiten 963-969, CSAPO, A.I. ET AL 'Prevention of implantation by antiprogesterone'
- THE NEW ENGLAND JOURNAL OF MEDICINE, Bd. 316, Nr. 4, 1987 Seiten 187-191, NIEMAN, L.K. ET AL 'The progesterone antagonist RU 486'
- HUMAN REPRODUCTION, Bd. 9, Nr. 2, Juni 1994 Seiten 11-21, LEBEAU, M.-C. ET AL 'Steroid antagonists and receptor-associated proteins'
- EUROPEAN JOURNAL OF OBSTETRICS, GYNAECOLOGY AND REPRODUCTIVE BIOLOGY, Bd. 4, Nr. 5, 1975 Seiten 161-166, BAULIEU, E.-E. 'Antiprogesterone effect and midcycle (periovulatory) contraception'

## Beschreibung

Die vorliegende Erfindung betrifft die Verwendung mindestens einer am Progesteronrezeptor gegenüber Progesteron antagonistischen Verbindung (nachfolgen kompetitiven Progesteronantagonist genannt) zur Herstellung eines Arzneimittels zur bedarfsorientierten weiblichen Fertilitätskontrolle ("Bedarfs-Pille"), welches unabhängig vom Zeitpunkt des Menstruationszyklus angewandt werden kann, in einer einmalig zu verabreichenden Dosiseinheit, in einer Dosierung, die eine Nidationshemmung, aber keine Ovulationshemmung bewirkt und einer wirkequivalenten Menge von 1 bis 400 mg Onapriston entspricht..

Bereits auf der ganzen Welt hat sich der Gebrauch von oralen Kontrazeptiva zu einem gesellschaftlichen Faktor entwickelt, der nicht mehr wegzudenken ist.
Besonders unter dem Aspekt der sich nach wie vor rasant entwickelnden Weltbevölkerung ist eine Weiterentwicklung der bislang bewährten Methoden zur Fertilitätskontrolle unbedingt erforderlich.

Der Einsatz von kompetitiven Progesteronantagonisten in der weiblichen Fertilitätskontrolle wird sowohl bei diversen Tierspezies als auch am Menschen schon seit einigen Jahren diskutiert, wie den nachfolgend aufgeführten Publikationen entnommen werden kann, wobei insbesondere der Einsatz von RU 486 (11-β-[4-N,N-(dimethylamino)phenyl]-17-β-hydroxy-17-α-propinyl-estra-4,9(10)-dien-3-on; EP-A-0057115) in diesem Zusammenhang aufgeführt wurde:
Collins et al., Blockade of the spontaneous mid-cycle gonadotropin surge in monkeys by RU 486; A progesterone antagonist or agonist. J. Cl. Metab., 63: 1270-1276 (1986);
Croxatto, H.B., Salvatierra 1990 Cyclic use of antigestagens for fertility control. IIIrd International Symposium on Contaception, Heidelberg, June 19-23, 1990;
Danford et al., Contraceptive potential of RU 486 by ovulation inhibition. III. Preliminary observations on once weekly administration. Contraception 40: 195-200 (1989);
Kekkonen et al., Lähteoenmäki P 1990 Interference with ovulation by sequential treatment with the antiprogesterone RU 486 and synthetic progestin. Fertil Steril [Fertile Sterile] 53: 4747 (1990);
Puri et al., Gonadal and pituitary responses to progesterone antagonist ZK 98 299 during the follicular phase of the menstral cycle in bonnet monkeys. Contraception 39(2): 227-243 (1989);
Puri et al., Contraceptive potential of a progesterone antagonist ZK 98 734: Effect on folliculogenesis, ovulation and corpus luteum function in bonnet monkeys. In Moudgal et al., (eds) (1990).

Hierbei ist zu erwähnen, daß die Dosierung, welche einen ovulationsinhibierenden Effekt zeigt, sehr stark von dem eingesetzten kompetitiven Progesteronantagonisten abhängt.

Die Klasse von 11β-Aryl- oder 11β, 19-Arylen-substituierten Steroiden wird pharmakologisch nach ihrem stark progesteron- bzw. glukocortikoid-antagonistischen Effekt unterschieden. So kann RU 468 einerseits für einen therapeutisch induzierten Schwangerschaftsabbruch (die humane abortive Dosis in Kombination mit einem Prostaglandin liegt bei 200-600 mg; EP-A 0 139 608), andererseits aber auch über seine antagonistische Wirkung am Glucocortikoid-Rezeptor zur Therapie des Cushing-Syndroms eingesetzt werden.

Eine andere Möglichkeit der Verwendung kompetitiver Progesteronantagonisten für die weibliche Fertilitätskontrolle, die sogenannte "LH+2"-Behandlung, wird von Swahn et al. [The effect of RU 486 administration during the early luteal phase on bleeding pattern, hormonal parameters and endometrium, Human Reproduction 5(4): 402-408 (1990)] vorgeschlagen, indem 2 Tage nach dem Anstieg des luteinisierenden Hormons (LH) im Menstruationszyklus der Frau (das ist im allg. am Tag 14, 15 oder 16) einmalig eine ovulationshemmende RU 486-Dosiseinheit verabreicht wird (luteale Kontrazeption).
Allerdings besitzt dieses Verfahren keine praktische Bedeutung, da die einfache und genaue zeitliche Bestimmung des LH-Peaks immer noch ein Problem darstellt.

Von Glasier et al. [Mifepristone (RU 486) compared with high-dose estrogen and progestogen for emergency postcoital contraception, The New England J. of Med. 327: 1041-1044 (1992)] wird auch die Verwendung von RU 486 für die postkoitale Kontrazeption (emergency postcoital contraception) beschrieben. Die Methode zeigt neben einer hohen Wirksamkeit ein geringes Ausmaß von Nebenwirkungen. Bei einem hohen Prozentsatz der Frauen dieser Studie trat eine Verlängerung des Zyklus auf. Dieser Effekt ist primär auf die antiovulatorische Wirkung von RU 486 zurückzuführen.

Des weiteren wird in WO 93/23020 beschrieben, daß kompetitive Progesteronantagonisten in einer Dosis, die sowohl unterhalb der abortiven als auch ovulationsinhibierenden Dosierung liegt, zur weiblichen Fertilitätskontrolle verwendet werden können. Allerdings ist hier eine im allgemeinen wöchentliche, bzw. mehrfache und damit regelmäßige Applikation erforderlich, um die gewünschte Wirkung zu erzielen.

Ebenso beschreibt die EP-A 0 219 447, welche Effekte die tägliche Gabe eines Progesteronantagonisten während der follikulären, bzw. optional auch der lutealen Phase des weiblichen Zyklus in einem Zeitraum von bis zu 4 Tagen in einer Dosierung von 10-200 mg bezüglich des endometrialen Differenzierungszustandes auslöst. Die hierbei resultierenden Veränderungen am Endometrium werden hinsichtlich des Nidationszeitpunktes für die invitro-Fertilisation genutzt.

Von Batista et al. [Daily administration of the progesterone antagonist RU 486 prevents implantation in the cycling guinea pig. Am. J. Obstet. Gynecol. 165: 82-86 (1991)] wird auch die Verwendung von RU 486 für die weibliche Fertilitätskontrolle beschrieben, welche durch tägliche Einnahme, präkoital und den gesamten weiteren Zyklus hindurch, in einer ovulationshemmenden Dosis die Nidation beim Meerschweinchen verhindert.

Von Kawano et al. [Effect of RU 486 on Glycogen Metabolism in Endometrium. Acta Obstetrica et Gynaecologica Japonica, 41: 1507-1511, (1989)] wird am Rattenmodell der Einfluß von RU 486 in einer Dosierung von 30 mg/kg Körpergewicht auf den endometrialen Glykogen-Metabolismus beschrieben, so daß eine erfolgreiche Eiimplantation gestört wird. Die Applikation erfolgt allerdings am Tag 2 oder 4 der Schwangerschaft.

Es wurde nunmehr gefunden, daß überraschenderweise bereits durch die einmalige, lediglich bedarfsweise Gabe eines kompetitiven Progesteronantagonisten (in subovulationshemmender Dosis) eine Nidation sicher verhindert werden kann und insofern ein neuartiges orales Kontrazeptivum zur Verfügung steht.

Bisher war die Verwendung von kompetitiven Progesteronantagonisten für die Kontrazeption nur durch eine mehrfache, regelmäßige Einnahme möglich.
Auch die bisherige Verwendung nach EP-A 0 219 447 steht zu der vorliegenden Erfindung in Kontrast, denn hier sollte eine Nidation durch die Progesteronantagonist-Gabe gerade erst ermöglicht werden.

Zweckmäßigerweise wird erfindungsgemäß ein sogenannter dissoziierter, kompetitiver Progesteronantagonist eingesetzt.

Unter einem dissoziierten, kompetitiven Progesteronantagonisten soll hierbei eine Substanz verstanden werden, die ihre Wirkung in der eingesetzten Dosierung nicht über die Hypothalamus-Ovarium-Achse im Sinne einer Ovulationshemmung (zentrale Wirkung) entfaltet, sondern einen ausschließlich auf das Endometrium begrenzten, lokalen Effekt (peripher selektive Wirkung) zeigt.

Bei einer bestimmtem Schwellendosis werden bereits Veränderungen am Endometrium beobachtet, während die Ovulation nicht gehemmt wird. Der Quotient aus ovulationshemmender und implantationsinhibierender Dosis (Dissozioationsfaktor) kann als ein Maß für die Dissoziation dienen. Er variiert je nach Spezies.

Alle bisher untersuchten kompetitiven Progesteronantagonisten zeigen bei der Ratte und am Primaten eine Dissoziation zwischen zentralen und endometrialen Effekten. Das Ausmaß dieser Dissoziation ist substanzabhängig. Für einen erfindungsgemäß zu verwendenden dissoziierten kompetitiven Progesteronantagonisten (ermittelt an der Ratte nach peroraler Applikation) sollte der Dissoziationfaktor vorzugsweise ungefähr bei 30 oder darüber liegen.

RU 486 ist ein Beispiel für eine wenig dissoziierte Substanz am Primaten. Es hemmt die Ovulation bereits bei niedrigen Dosen und führt dadurch zu Zyklusstörungen.
Onapriston (11β-[4-N,N-(Dimethylamino)phenyl]-17α-hydroxy-17β-(3-hydroxypropyl)-13α-methyl-4,9(10)-gonadien-3-on; EP-A 0 129 499) ist ein Beispiel für eine stark dissozierte progesteronantagonistische Verbindung, die erst bei extrem hohen Dosen die Ovulation an Primaten hemmt.
Die endometrialen Effekte von RU 486 und Onapriston treten dagegen bei vergleichbaren Dosen auf. Diese Dissoziation zwischen zentralem und endometrialem Effekt macht es möglich, bei Verwendung eines dissoziierten kompetitiven Progesteronantagonisten für die Zwecke der vorliegenden Erfindung dessen Dosis zu erhöhen und so eine wirksame Implantationshemmung nach Einmalanwendung zu gewährleisten.

Insofern ist mit der erfindungsgemäßen Lösung auch eine weitere, an moderne Pharmaka zu stellende Forderung erfüllt, daß nämlich durch eine lokal differenzierte Wirkungsweise eine systemische Belastung des Organismus durch Einmalgabe vermieden wird.

Durch die bisherigen Ausführungen wird deutlich, daß eine bedarfsorientierte orale Fertilitätskontrolle bis heute nicht möglich war.

Gerade für Frauen, die sich nicht zu der bislang erforderlichen, regelmäßigen Einnahme von oralen Kontrazeptiva entschließen konnten oder auch aus anderen, z.B. medizinischen Gründen nicht die Möglichkeit hatten, diese Form der Fertilitätskontrolle zu wählen, bietet sich nunmehr die Alternative, sich für die einmalige, bedarfsorientierte Einnahme eines oralen Kontrazeptivum zu entscheiden, ohne ihren Organismus einer den gesamten Menstruationszyklus fortdauernden hormonellen Kontrolle auszusetzen.

Die vorliegende Erfindung besitzt des weiteren wesentliche Vorteile, die nicht zuletzt in der niedrigen Dosierung des Wirkstoffes begründet liegen.
So wird der weibliche Menstruationszyklus in keiner Weise in seiner Zyklizität beeinträchtigt (wie durch ovulationshemmende Substanzen verursacht) und der Organismus nicht durch unnötig hohe Mengen des kompetitiven Progesteronantagonisten belastet.

Der Gebrauch eines solchen Progesteronantagonisten bietet ferner eine sichere Empfängnisverhütung, d.h. die einmalige Einnahme eines derartigen Medikamentes verhindert die Einnistung der Blastozyste.

Die kompetitiven Progesteronantagonisten werden gemäß vorliegender Erfindung in Mengen von im allgemeinen einmalig 1 - 400 mg Onapriston oder einer biologischen Äquivalentdosis eines anderen kompetitiven Progesteronantagonisten verwendet.

Die Behandlung mit dem kompetitiven Progesteronantagonisten wird in der Regel durch die einmalige, bedarfsorientierte, d.h. im allgemeinen prä- oder auch postkoitale Einnahme einer Tagesdosiseinheit unabhängig vom Zykluszeitpunkt vorgenommen.
Die präkoitale Einnahme des erfindungsgemäß hergestellten Medikaments ist bevorzugt.
Der Zeitpunkt der Einnahme kann bis zu 6 Stunden vor oder bis zu 24 Stunden nach dem Koitus liegen.
Die Einnahme ist dabei zu jedem Zykluszeitpunkt, an dem eine kontrazeptive Maßnahme erforderlich, angeraten oder gewünscht ist, möglich.

Als kompetitive Progesteronantagonisten sind erfindungsgemäß alle Verbindungen geeignet, die eine entsprechend charakteristische Affinität zum Progesteronrezeptor zeigen und eine Endometrium-selektive Wirkung aufweisen. Derartige Progesteronantagonisten zeigen in der eingesetzten Dosierung eine Nidationshemmung ohne eine Hemmung der Ovulation zu bewirken. So kommen beispielsweise die folgenden Progesteronantagonisten infrage:
11β-[4-N,N-(Dimethylamino)phenyl]-17α-hydroxy-17β-(3-hydroxypropyl)-13α-methyl-4,9(10)-gonadien-3-on (EP-A 0 129 499),
11β-(4-Acetylphenyl)-17β-hydroxy-17α-(3-hydroxyprop-1(Z)-enyl)-4,9(10)-estradien-3-on (EP-A 0 190 759),
11β,19-[4-(Cyanphenyl)-o-phenylen]-17β-hydroxy-17α-(3-hydroxyprop-1(Z)enyl)-4-androsten 3-on,
11β,19-[4-(3-Pyridinyl)-o-phenylen]-17β-hydroxy-17α-(3-hydroxyprop-1(Z)-enyl)-4-androsten 3-on (beide WO-A 93/23020)
17α-Hydroxy-17β-(3-hydroxypropyl)-11β-[4-(1-methylethenyl)phenyl]-13α-estra-4,9-dien-3-on,
11β-[4-(3-Furanyl)phenyl]-17α-hydroxy-17β-(3-hydroxypropyl)-13α-estra-4,9-dien-3-on (beide EP-A 0 349 481),
(Z)-11β-[4-(Dimethylamino)phenyl]-17β-hydroxy-17α-(3-hydroxyprop-1-enyl)estr-4-en-3-on (EP-A 0 404 283),
11β-[4-[[(Acetyloxy)imino]methyl]phenyl]-17β-methoxy-17α-(methoxymethyl)estra-4,9-dien-3-on (EP-A 0 648 778, EP-A 0 648 779),
11β-[4-[[[[(Ethoxycarbonyl)oxy]imino]methyl]phenyl]-17β-methoxy-17α-(methoxymethyl)estra-4,9-dien-3-on (EP-A 0 648 778, EP-A 0 648 779),
11β-[4-(Acetyl)phenyl]-19,24-dinor-17,23-epoxy-17α-chola-4,9,20-trien-3-on,
(11β,17α)-11-[4-(Acetyl)phenyl]-17,23-epoxy-19,24-dinorchola-4,9,20-trien-3-on.

Die kompetitiven Progesteronantagonisten können beispielsweise lokal, topisch, enteral oder parenteral appliziert werden.

Für die bevorzugte orale Applikation kommen insbesondere Tabletten, Dragees, Kapseln, Pillen, Suspensionen oder Lösungen infrage, die in üblicher Weise mit den in der Galenik gebräuchlichen Zusätzen und Trägersubstanzen hergestellt werden können.
Die einmalig zu verabreichende orale Dosierungseinheit enthält etwa 2 bis 200 mg Onapriston oder die biologische Äquivalentdosis (wirkequivalente Menge) eines anderen dissoziierten kompetitiven Progesteronantagonisten.
Als wirkequivalente Menge gilt die Dosis, die beim Cynomolgus-Affen nach einer einmaligen Behandlung am Tag 22 des Zyklus die vorzeitige Menstruation induziert, die Ovulation, wenn vor dem LH-Peak (LH - 2) einmalig verabreicht, aber nicht hemmt.

Vor allem bei der bevorzugten oralen Applikation des erfindungsgemäß hergestellten Arzneimittels ist es wünschenswert, daß eine verzögerte Freisetzung des jeweiligen Wirkstoffes stattfindet. Dadurch soll sichergestellt werden, daß es nicht zu einer verzögerten Implantation des befruchteten Eies kommen kann.
Eine verzögerte Freisetzung des kompetitiven Progesteronantagonisten läßt sich beispielsweise durch Formulierung der oral zu verabreichenden Dosierungseinheit als Matrixtablette oder durch Versehen der oral zu verabreichenden Dosierungseinheit mit einem retardierenden Überzug bewerkstelligen, wie dies dem Fachmann ohne weiteres bekannt ist. Es kann auch der zur Herstellung des erfindungsgemäßen Arzneimittels verwendete kompetitive Progesteronantagonist durch Derivatisierung, beispielsweise durch Veresterung einer freien Hydroxygruppe in einem wirksamen Vorläufer, eine längere Halbwertszeit als diese Vorstufe aufweisen. Dadurch wird ebenfalls eine länger anhaltende Wirkung erreicht. Dieses Prinzip ist beispielsweise in den in der EP-A 0186 834 beschriebenen Estern des 11β-[4-N,N-(Dimethylamino)phenyl]-17α-hydroxy-17β-(3-hydroxypropyl)-13α-methyl-4,9(10)-gonadien-3-ons (Onapriston) verwirklicht, die deshalb im Rahmen vorliegender Erfindung ebenfalls Verwendung finden können. Als Vertreter seien genannt:
17β-(3-Acetoxypropyl)-11β-[4-N,N-(dimethylamino)phenyl]-17α-hydroxy-13α-methyl-4,9(10)-gonadien-3-on und
17β-(3-Benzoyloxypropyl)-11β-[4-N,N-(dimethylamino)phenyl]-17α-hydroxy-13α-methyl-4,9(10)-gonadien-3-on.

Für die lokale oder topische Anwendung stehen außerdem beispielsweise Vaginalzäpfchen oder transdermale Systeme wie Hautpflaster zur Verfügung.
Die einmalig zu verabreichende Dosierungseinheit enthält für diese spezielle Applikationsform eine solche Menge Onapriston oder eine biologische Äquivalentdosis eines anderen Progesteronantagonisten, daß über einen Zeitraum von 4 bis 72 Stunden 1 bis 400 mg dieses kompetitiven Progesteronantagonisten freigesetzt werden.

Die nachstehenden Beispiele sollen die Formulierung eines kompetitiven Progesteronantagonisten erläutern, die im Rahmen der vorliegenden Erfindung für die Anwendung von besonderer Relevanz ist.

### Beispiel 1

Zusammensetzung einer Tablette mit 10,0 mg 11β-[(4-N,N-Dimethylamino)-phenyl]-17α-hydroxy-17β-(3-hydroxypropyl)-13α-methyl-4,9-gonadien-3-on zur oralen Applikation

| | |
|---|---|
| 10,0 mg | 11β-[(4-N,N-Dimethylamino)-phenyl]-17α-hydroxy-17β-(3-hydroxypropyl)-13α-methyl-4,9-gonadien-3-on |
| 140,5 mg | Laktose |
| 69,5 mg | Maisstärke |
| 2,5 mg | Poly-N-vinylpyrrolidon |
| 2,0 mg | Aerosil |
| 0,5 mg | Magnesiumstearat |
| 225,0 mg ======= | Gesamtgewicht der Tablette |

### Beispiel 2

Zusammensetzung einer Tablette mit 50,0 mg 11β,19-[4-(Cyanphenyl)-o-phenylen]-17β-hydroxy-17α-(3-hydroxyprop-1(Z)-enyl)-4-androsten 3-on zur oralen Applikation

| | |
|---|---|
| 50,0 mg | 11β, 19-[4-(Cyanphenyl)-o-phenylen]-17β-hydroxy-17α-(3-hydroxyprop-1(Z)-enyl)-4-androsten 3-on |
| 140,5 mg | Laktose |
| 69,5 mg | Maisstärke |
| 2,5 mg | Poly-N-vinylpyrrolidon |
| 2,0 mg | Aerosil |
| 0,5 mg | Magnesiumstearat |
| 265,0 mg ======= | Gesamtgewicht der Tablette |

Die Tabletten werden in bekannter Weise auf einer Tablettenpresse hergestellt und mit einem retardierenden Überzug versehen.

### Pharmakologische Beobachtungen

Die Eignung, insbesondere dissoziierter, kompetitiver Progesteronantagonisten zur Herstellung eines Arzneimittels zur bedarfsorientierten weiblichen Fertilitätskontrolle ("Bedarfs-Pille"), welches einmalig in einer nicht-ovulationsinhibierenden Dosierung des kompetitiven Progesteronantagonisten verabreicht werden kann, ergibt sich aus den nachfolgend beschriebenen tierexperimentellen und human-pharmakologischen Beobachtungen:

Zyklische weibliche Affen (Körpergewicht ca. 4-5 kg) werden einmalig über 3 Zyklen in der späten Follikulärphase des Zyklus (1-2 Tage vor der Ovulation) mit 10 bzw. 30 mg/kg s.c. bzw. 30 mg/kg p.o. Onapriston behandelt.
Anschließend werden die Affen angepaart. Aufgrund der Progesteronbestimmung in der Lutealphase wurde festgestellt, daß die Ovulation stattgefunden hat und daß der Verlauf der Lutealphase normal war. Die Blutung (Menstruation) trat erwartungsgemäß am Tag 27-31 des Zyklus auf. Bei den behandelten Tieren wurde keine, hingegen bei den Kontrolltieren, die nur mit Vehikel behandelt wurden, nach der Anpaarung eine Schwangerschaft festgestellt.

Andere Affen werden in der frühen Lutealphase (Tag 1-3 nach der Ovulation) über 3 Zyklen einmalig mit 10 bzw. 30 mg Onapriston/kg s.c. bzw. 30 mg/kg p.o. behandelt. Nach erfolgter Anpaarung waren auch nach diesem Behandlungsschema bei den mit Onapriston behandelten Tieren keine Schwangerschaften festzustellen.

Probandinnen, die einen normalen Zyklus aufweisen, werden mit 100 bzw. 400 mg Onapriston am 2. Tag vor der Ovulation (LH-2) oral einmalig behandelt. Die Hormonprofile (Estradiol, Progesteron, LH) belegen, daß die Ovulation nicht gehemmt war. Eine wesentliche Verkürzung bzw. Verlängerung des Zyklus konnte nicht nachgewiesen werden.

Andere Probandinnen werden mit 100 bzw. 400 mg Onapriston 2 Tage nach der Ovulation (LH+2) einmalig oral behandelt. Anschließend wird 4 und 6 Tage nach der Ovulation eine Biopsie des Endometriums vorgenommen. Die Histologie ergab deutliche Veränderungen bei den behandelten Frauen im Sinne einer Desynchronisation des Endometriums.
Klinische Erfahrungen mit infertilen Frauen deuten darauf hin, daß gerade wegen derartiger Veränderungen des Endometriums eine erfolgreiche Implantation als unwahrscheinlich gilt und somit auch bei den Frauen der Behandlungsgruppe eine erfolgreiche Implantation nicht zu erwarten ist.

## Patentansprüche

1. Verwendung mindestens einer am Progesteronrezeptor gegenüber Progesteron antagonistischen Verbindung zur Herstellung eines Arzneimittels zur bedarfsorientierten weiblichen Fertilitätskontrolle, welches unabhängig vom Zeitpunkt des Menstruationszyklus angewandt werden kann, in einer einmalig zu verabreichenden Dosiseinheit, in einer Dosierung, die eine Nidationshemmung, aber keine Ovulationshemmung bewirkt und einer wirkequivalenten Menge von 1 bis 400 mg Onapriston entspricht.

2. Verwendung nach Anspruch 1, **dadurch gekennzeichnet, daß** die Verbindung aus der Gruppe der folgenden Verbindungen ausgewählt ist:
11β-[4-N,N-(Dimethylamino)phenyl]-17α-hydroxy-(3-hydroxypropyl)-13α-methyl-4,9(10)-gonadien-3-on,
11β-(4-Acetylphenyl)-17β-hydroxy-17α-(3-hydroxyprop-1(Z)-enyl)-4,9(10)-estradien-3-on,
11β,19-[4-(Cyanphenyl)-o-phenylen]-17β-hydroxy-17α-(3-hydroxyprop-1(Z)-enyl)-4-androsten 3-on,
11β, 19-[4-(3-Pyridinyl)-o-phenylen]-17β-hydroxy-17α-(3-hydroxyprop-1(Z)-enyl)-4-androsten 3-on,
17α-Hydroxy-17β-(3-hydroxypropyl)-11β-[4-(1-methylethenyl)phenyl]-13α-estra-4,9-dien-3-on,
11β-[4-(3-Furanyl)phenyl]-17α-hydroxy-17β-(3-hydroxypropyl)-13α-estra-4,9-dien-3-on,
(Z)-11β-[4-(Dimethylamino)phenyl]-17β-hydroxy-17α-(3-hydroxyprop-1-enyl)estr-4-en-3-on
11β-[4-[[(Aetyloxy)imino]methyl]phenyl]-17β-methoxy-17α-(methoxymethyl)estra-4,9-dien-3-on
11β-[4-[[[[(Ethoxycarbonyl)oxy]imino]methyl]phenyl]-17β-methoxy-17α-(methoxymethyl)estra-4,9-dien-3-on
11β-[4-(Acetyl)phenyl]-19,24-dinor-17,23-epoxy-17α-chola-4,9,20-trien-3-on,
(11β,17α)-11-[4-(Acetyl)phenyl]-17,23-epoxy-19,24-dinorchola-4,9,20-trien-3-on.

3. Verwendung nach Anspruch 1, **dadurch gekennzeichnet, daß** die Verbindung aus der Gruppe der folgenden Verbindungen ausgewählt ist:
17β-(3-Acetoxypropyl)-11β-[4-N,N-(dimethylamino)phenyl]-17α-hydroxy-13α-methyl-4,9(10)-gonadien-3-on,
17β-(3-Benzoyloxypropyl)-11β-(4-N, N-(dimethylamino)phenyl]-17α-hydroxy-13α-methyl-4,9(10)-gonadien-3-on.

4. Verwendung nach Anspruch 1, **dadurch gekennzeichnet, daß** die Verbindung in dem Arzneimittel zur Applikation in lokaler, topischer, enteraler oder parenteraler Weise hergerichtet ist.

## Claims

1. Use of at least one competitive progesterone antagonist in the manufacture of an on-demand female fertility control medicament which can be used at any point in the menstrual cycle, in a single dose, in a dosage which effects nidation inhibition but not ovulation inhibition and corresponds to a functionally equivalent amount of 1 to 400 mg of onapristone.

2. Use according to Claim 1, **characterized in that** the competitive progesterone antagonist is selected from the group of the following compounds:
11β-[4-N,N-(dimethylamino)phenyl]-17α-hydroxy-17β-(3-hydroxypropyl)-13α-methyl-4,9(10)-gonadien-3-one,
11β-[4-acetylphenyl]-17β-hydroxy-17α-(3-hydroxyprop-1(Z)-enyl)-4,9(10)-estradien-3-one,
11β,19-[4-(cyanophenyl)-o-phenylene]-17β-hydroxy-17α-(3-hydroxyprop-1(Z)-enyl)-4-androsten-3-one,
11β, 19-[4-(3-pyridinyl)-o-phenylene]-17β-hydroxy-17α-(3-hydroxyprop-1(Z)-enyl)-4-androsten-3-one,
17α-hydroxy-17β-(3-hydroxypropyl)-11β-[4-(1-methylethenyl)phenyl]-13α-estra-4,9-dien-3-one,
11β-[4-(3-furanyl)phenyl]-17α-hydroxy-17β-(3-hydroxypropyl)-13α-estra-4,9-dien-3-one,
(Z)-11β-[4-(dimethylamino)phenyl]-17β-hydroxy-17α-(3-hydroxyprop-1-enyl)estr-4-en-3-one,
11β-[4-[[(acetyloxy)imino]methyl]phenyl]-17β-methoxy-17α-(methoxymethyl)estra-4,9-dien-3-one,
11β-[4-[[[[(ethoxycarbonyl)oxy]imino]methyl]phenyl]-17β-methoxy-17α-(methoxymethyl)estra-4,9-dien-3-one,
11β-[4-(acetyl)phenyl]-19,24-dinor-17,23-epoxy-17α-chola-4,9,20-trien-3-one, or (11β, 17α)-11-[4-(acetyl)-phenyl]-17,23-epoxy-19,24-dinorchola-4,9,20-triene-3-one.

3. Use according to Claim 1, **characterized in that** the competitive progesterone antagonist is selected from the group of the following compounds:
17β-(3-acetoxypropyl)-11β-[4-N,N-(dimethylamino)phenyl]-17α-hydroxy-13α-methyl-4,9(10)-gonadien-3-one, or
17β-(3-benzoyloxypropyl)-11β-[4-N,N-(dimethylamino)phenyl]-17α-hydroxy-13α-methyl-4,9(10)-gonadien-3-one.

4. Use according to Claim 1, **characterized in that** the competitive progesterone antagonist in the compound has been formulated for application in a local, topical, enteral or parenteral manner.

## Revendications

1. Utilisation d'au moins un composé antagoniste du récepteur de la progestérone par rapport à la progestérone, pour préparer un médicament destiné à la régulation de la fertilité féminine, orientée selon les besoins, qui peut être utilisé indépendamment de l'instant du cycle menstruel, dans le cadre d'une unité posologique devant être administrée en une fois, à une posologie qui conduit à une inhibition de la nidation, mais non pas à une inhibition de l'ovulation, et en une quantité dont l'action est équivalente à celle de 1 à 400 mg d'onapristone.

2. Utilisation selon la revendication 1, **caractérisée en ce que** le composé est choisi dans le groupe des composés suivants :
11β-[4-N,N-(diméthylamino)phényl]-17α-hydroxy-(3-hydroxypropyl)-13α-méthyl-4,9(10)-gonadiène-3-one,
11β-(4-acétylphényl)-17β-hydroxy-17α-(3-hydroxyprop-1(Z)-ényl)-4,9(10)-oestradiène-3-one,
11β,19-[4-(cyanophényl)-o-phénylène]-17β-hydroxy-17α-(3-hydroxyprop-1(Z)-ényl)-4-androstène-3-one,
11β,19-[4-(3-piridinyl)-o-phénylène]-17β-hydroxy-17α-(3-hydroxyprop-1(Z)-ényl)-4-androstène-3-one,
17α-hydroxy-17β-(3-hydroxypropyl)-11β-[4-(1-méthyléthényl) phényl]-13α-oestra-4,9-diène-3-one,
11β-[4-(3-furannyl)phényl]-17α-hydroxy-17β-(3-hydroxypropyl)-13α-oestra-4,9-diène-3-one,
(Z)-11β-[4-(diméthylamino)phényl]-17β-hydroxy-17α-(3-hydroxyprop-1-ényl)oestr-4-ène-3-one,
11β-[4-[[(acétyloxy)imino]méthyl]phényl]-17β-méthoxy-17α-(méthoxyméthyl)oestra-4,9-diène-3-one,
11β-[4-[[[[(éthoxycarbonyl)oxy]imino]méthyl]-phényl]-17β-méthoxy-17α-(méthoxyméthyl)oestra-4,9-diène-3-one,
11β-[4-(acétylphényl]-19,24-dinor-17,23-époxy-17α-chola-4,9,20-triène-3-one,
(11β-17α)-11-[4-(acétyl)phényl]-17,23-époxy-19,24-dinorchola-4,9,20-triène-3-one.

3. Utilisation selon la revendication 1, **caractérisée en ce que** le composé est choisi dans le groupe des composés suivants :
17β-(3-acétoxypropyl)-11β-[4-N,N-(diméthylamino)-phényl]-17α-hydroxy-13α-méthyl-4,9(10)-gonadiène-3-one,
17β-(3-benzoyloxypropyl)-11β-[4-N,N-(diméthylamino) phényl]-17α-hydroxy-13α-méthyl-4,9(10)-gonadiène-3-one,

4. Utilisation selon la revendication 1, **caractérisée en ce que** le composé est préparé dans le médicament destiné à une administration par voie locale, topique, entérale ou parentérale.
